# EUROPEAN PATENT SPECIFICATION

(11) **EP 0 687 168 B1**
(45) Date of publication and mention of the grant of the patent: **24.05.2000**
(21) Application number: 94906058.6
(22) Date of filing: 07.01.1994
(51) Int. Cl.: A61F 6/08

(54) **DISPOSABLE VAGINAL DEVICE**
VAGINAL-VORRICHTUNG ZUM EINMALIGEN GEBRAUCH
DISPOSITIF VAGINAL JETABLE

(30) Priority: 26.01.1993 US 9578
(43) Date of publication of application: 20.12.1995
(73) Proprietor: Veos Limited, St. Helier, Jersey JE1 1BJ, Channel Islands (GB)
(72) Inventor: LAVEAN, Michael, G., Saranac, MI 48881 (US); TLAPEK, Janet, M., Saranac, MI 48881 (US)
(74) Representative: Price, Nigel John King
(86) International application number: US9400288
(87) International publication number: WO9416652

(56) References cited:
- FR-A- 2 287 917
- GB-A- 1 430 533
- GB-A- 2 079 158
- US-A- 4 311 543
- US-A- 4 578 076
- US-A- 5 044 376

## Description

### BACKGROUND OF THE INVENTION

The present invention relates to a vaginal device according to the preamble of claim 1.

Devices which are intended to be inserted into the vagina are known for use as contraceptive barriers or as vehicles for the delivery of medicaments or spermicides. In the past, devices for the delivery of biologically active materials have included pessaries, vaginal suppositories and various devices composed of one or more reservoirs covered by a permeable surface through which the biologically active material may migrate or leave the device and then enter the body. One of the principal requirements for a device for delivering a biologically active substance to the body is that the device remain in place long enough to administer the desired dose.

Although vaginal devices have been known for some time, there is continuing development of their design and use around the world. For example, cervical caps are becoming increasingly popular for contraceptive use by the general population. Despite advances in the art, there remains a need for improved intra-vaginal devices capable of delivering a biologically active substance yet which are inexpensive and which can be self inserted. Preferably, the vaginal device is capable of acting as a multi-purpose delivery vehicle for two or more biologically active materials. There is also a need for a vaginal device which can be readily adapted for various uses and which has a long shelf life.

FR-A-2 287 917, upon which the preamble of claim 1 is based, discloses a vaginal ring device having a support ring with one or two pocket-shaped recesses extending circumferentially around the device to receive LTV-silicone elastomer rings containing a pharmacologically active substance. The device is manufactured into one piece by vulcanization.

GB-A-1 430 533 discloses a drug supporting anchor for insertion and retention in a body cavity of a mammal. The anchor has an elongated member formed by spaced coils which define a hollow core and support a slow-release drug to be administered in the body cavity.

GB-A-2 079 158 relates to an intravaginal device for animals that includes a body with a skin over at least part of the outer surface of the body, wherein the skin has at least one active ingredient, such as progesterone or oestrogen.

US-A-4 311 543 relates to a method of manufacturing disposable contraceptive intravaginal barriers. The barriers are made of plastic foam that is impregnated with a medicament that can be activated by contact with a small amount of aqueous solution.

US-A-4 578 076 discloses a medicated device for retention in the cervix or uterus that includes a T-shaped platform with a drug release attachment. The platform of the device also includes a rounded knob portion at the top and two flat slanting arms extending downwardly from the knob.

US-A-5 044 376 relates to a reusable intravaginal device, such as a diaphragm or cervical cap, which has an integral pad-retaining member or pouch shaped to hold foam pad which releases a solution of chemicals, such as Nonoxynol-9, over the vaginal opening of the cervix. The chemicals are dispensed during intercourse and designed to prevent pregnancy and sexually transmitted diseases.

The present invention represents a significant conceptual advance in the art relating to the way biologically active materials are introduced intra-vaginally. The new device of this invention is capable of being molded into parts which are assembled interchangeably. The present invention allows intra-vaginal, sustained delivery of biological active materials to treat a variety of sexually transmitted diseases, yeast infections, warts, ulcerations, lesions, endometriosis, hormone deficiency, etc. It will be apparent to those familiar with the art that there are a tremendous number of variations and combinations of biological active materials that could be delivered intra-vaginally in order to address a whole host of problems. For example: a preferred treatment for Chlamydia involves the administration of Tetracycline and Ampicillin for a ten day period. However, patients often fail to properly comply with the terms of medication for the ten day period. The present invention allows the delivery of Tetracycline and Ampicillin in combination in such a way that patient compliance can be removed as an issue.

In summary, the present invention provides devices including cervical caps, diaphragms, vaginal rings and other intra-vaginal devices designed for delivery of biologically active materials. Further understanding of the present invention will be had from the following disclosure and claims taken in conjunction with accompanying drawings.

### SUMMARY OF THE INVENTION

According to the present invention there is provided a vaginal device having a first member and a second member, at least one of said members comprising a releasable biologically active material, characterised in that said members are interchangeably and selectively snap-fit together.

The device is assembled by selectively attaching two or more parts and is used for the sustained delivery of one or more biologically active materials. The device is able to sustain delivery of a wide variety of drugs simultaneously. The parts can be located in a pharmacy so that a physician would be able to prescribe the device with the appropriate "snap ins" and the pharmacist would be able to assemble it prior to dispensing the device to the user.

The parts to be used can be manufactured of a biologically active loaded silicone based material and are snap-fit together. Thus, the device is composed of two or more parts designed to be selectively snapped together prior to being inserted by the user. The parts of the current invention can be made in such a way as to promote the greatest possible amount of interchangeability.

In a preferred embodiment, the invention will be composed of a cervical cap with a plurality of grooves allowing a plurality of snap-in rings to be added prior to use. Alternative preferred embodiments include a diaphragm vaginal ring, vaginal disc or vaginal suppository, upon which there would be a plurality of snap-in parts making a whole unit prior to use. Still further preferred alternative embodiments are two or more parts that when selectively snapped together, would form a cervical cap, diaphragm, vaginal disc or a vaginal suppository. Combinations of the above alternatives can also be used.

### BRIEF DESCRIPTION OF THE DRAWINGS

Figure 1 is a perspective view of a preferred embodiment of a cervical cap of the present invention;
Figure 2 is a sectional view, broken away, taken along line 2-2 in Figure 1;
Figures 3 - 5 are sectional views analogous to Figure 2 but showing alternative preferred embodiments of rims of cervical caps of the present invention;
Figure 6 is an exploded view in perspective, showing another alternative preferred embodiment of a cervical cap of the present invention;
Figure 7 is a sectional view, broken away, taken along line 7-7 in Figure 6;
Figure 8 is an exploded view, in perspective, analogous to Figure 6 but showing yet another alternative preferred embodiment of a cervical cap of the present invention;
Figure 9 is a sectional view, broken away, taken along line 9-9 in Figure 8;
Figures 10 - 11 are sectional views analogous to Figure 9 but showing further alternative preferred embodiments of cervical caps of the present invention;
Figure 12 is a perspective view showing a preferred embodiment of a vaginal ring of the present invention;
Figure 13 is a sectional view, broken away, taken along line 13-13 in Figure 12;
Figures 14 - 16 are exploded views, in perspective, of alternative preferred embodiments of vaginal suppositories of the present invention.
Figure 17 is a perspective view of a preferred embodiment of a vaginal ring of the present invention.
Figure 18 is a sectional view, partly in perspective and broken away, taken along lines 18-18 in Figure 17.
Figures 19 - 20 are perspective views of alternative preferred embodiments of vaginal devices of the present invention.
Figure 21 is a perspective view of a preferred embodiment of a vaginal ring of the present invention.
Figure 22 is a perspective view of a preferred embodiment of a vaginal suppository of the present invention.
Figure 23 is a perspective view of a preferred embodiment of a diaphragm of the present invention.
Figure 24 is a sectional view taken along line 24-24 in Figure 23 and broken away.
Figure 25 is a perspective view of a preferred embodiment of an alternative diaphragm of the present invention.
Figure 26 is a sectional view, broken away, taken along line 26-26 in Figure 25.
Figures 27 - 28 are sectional views, broken away, analogous to Figure 26 but showing alternative embodiments of diaphragms of the present invention.

### DETAILED DESCRIPTION OF THE PREFERRED EMBODIMENT

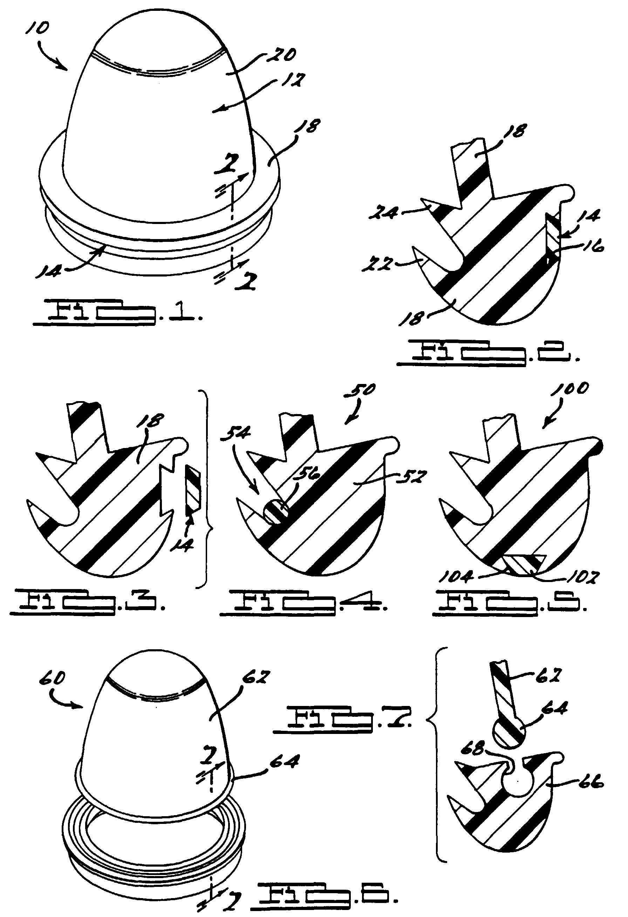

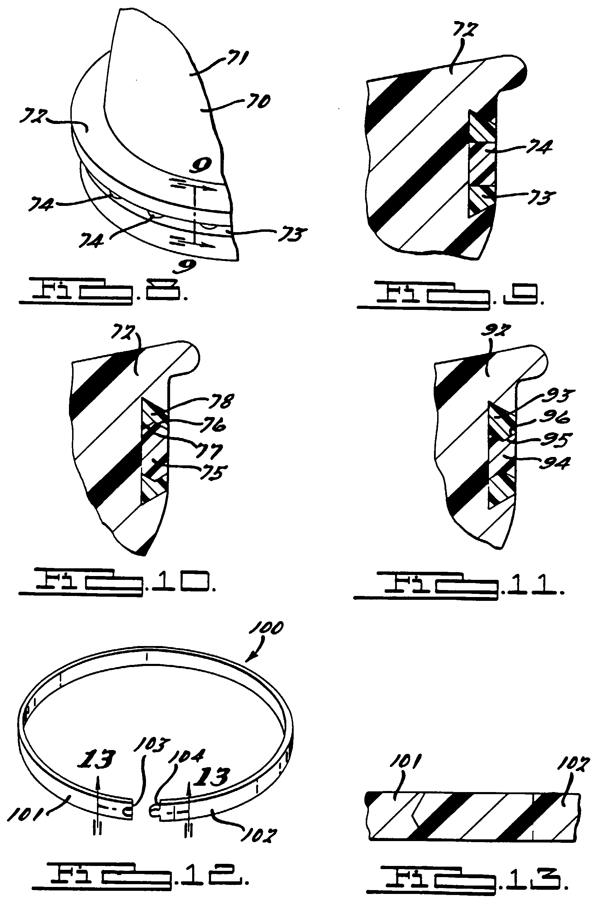

Now referring to Figure 1, a preferred embodiment of a vaginal device of the present invention is shown in perspective view and indicated generally by the numeral 10. Vaginal device 10 is a cervical cap which, broadly speaking, has a dome 12 with an annular ring 14 retained thereon. As best shown in Figure 2, annular ring 14 is snap-fit into, and retained by, groove 16 in annular rim 18 of dome 12.

Dome 12 is generally thimble-shaped and sized to fit over the cervix in a manner conventional for cervical caps. Annular rim 18 is integrally molded with thin, flexible non-resilient cap portion 20 of dome 12. Annular rim 18 has generally inwardly and upwardly, as viewed in Figure 2, directed ribs 22 and 24 for gripping the cervix wall to retain vaginal device 10 in position over the cervix. Groove 16 extends around rim 18 on the radially outside face of annular rim 18. Groove 16 and annular ring 14 are shaped in cross-section and sized so that annular ring 14 in snappingly retained groove 16.

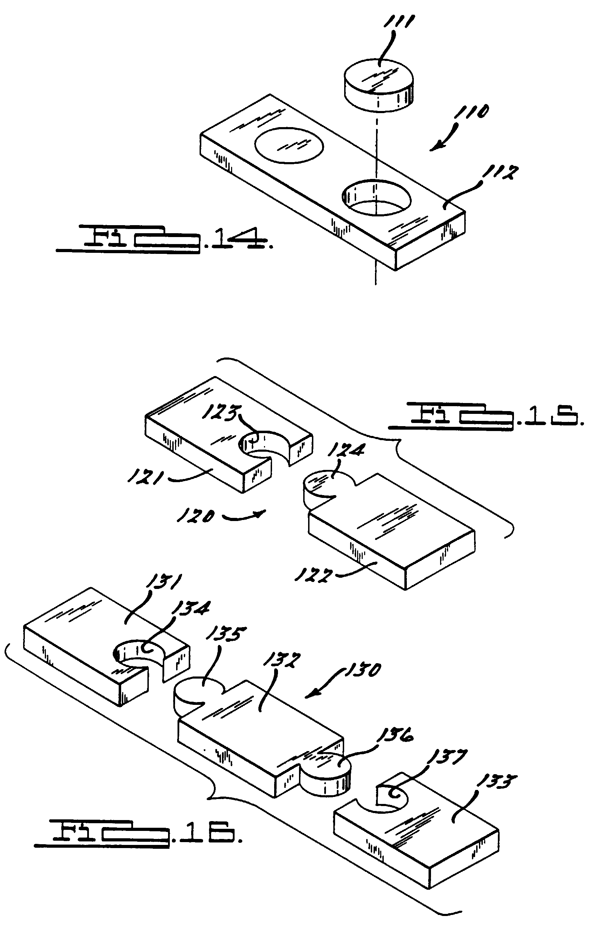

As is illustrated in the exploded view of Figure 3, it is contemplated that annular ring 14 will be made as an element separate from rim 18 of dome 12 and then assembled with dome 12 to provide a complete vaginal device 10, a cervical cap.

It is contemplated that dome 12 will preferably be made of a non-resilient flexible material, such as a silicone based material, and that annular ring 14 will comprise a similar or the same material but be loaded, i.e., impregnated, with a desired biologically active material such as a medicament which will be released therefrom in an amount effective to achieve its purpose during use.

Types of silicone based materials suitable for use herein are known in the art and include high-consistency and low-consistency silicone-based elastomers prepared using a variety of well-known methods, e.g., platinum-cured systems, selected for compatibility with biological tissue and particular active ingredients being released by the device. An example of a biologically-active agent that could be released by the device is nonoxynol-9, commonly known as "N-9", an anti-viral compound often used with contraceptive devices. The elastomer can be loaded with the active agent in a manner appreciated by those skilled in the art that incorporates the agent in an excipient matrix with the elastomer, providing sustained release of the agent from the matrix.

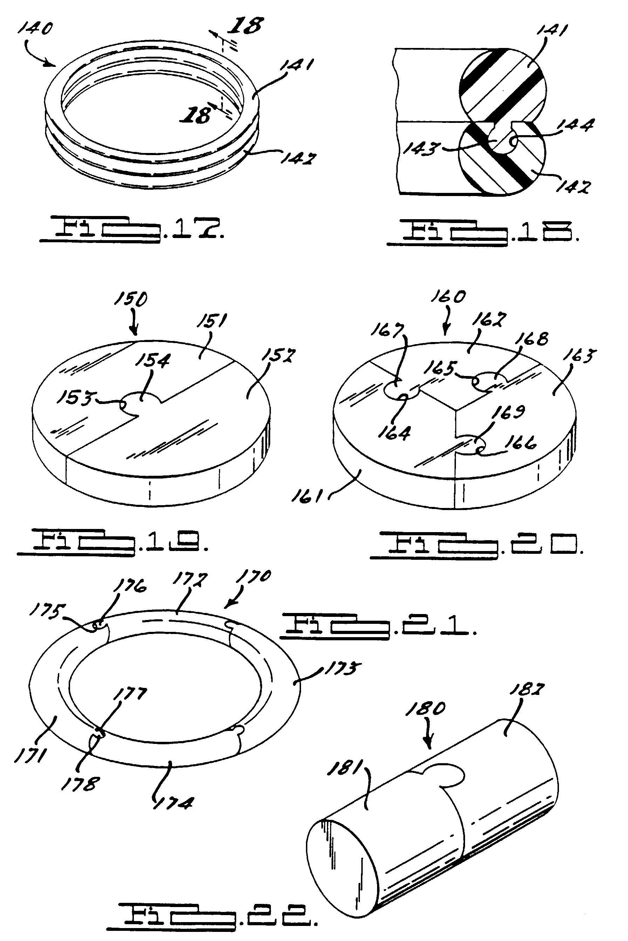

Further alternative embodiments of vaginal device 10 are illustrated the remaining Figures 4 - 28. The vaginal device 50 of Figure 4 is of a construction analogous to vaginal device 10 of Figures 1-3 except that annular rim 52 has groove 54 carrying O-ring 56 which comprises a flexible material loaded or impregnated with medicament.

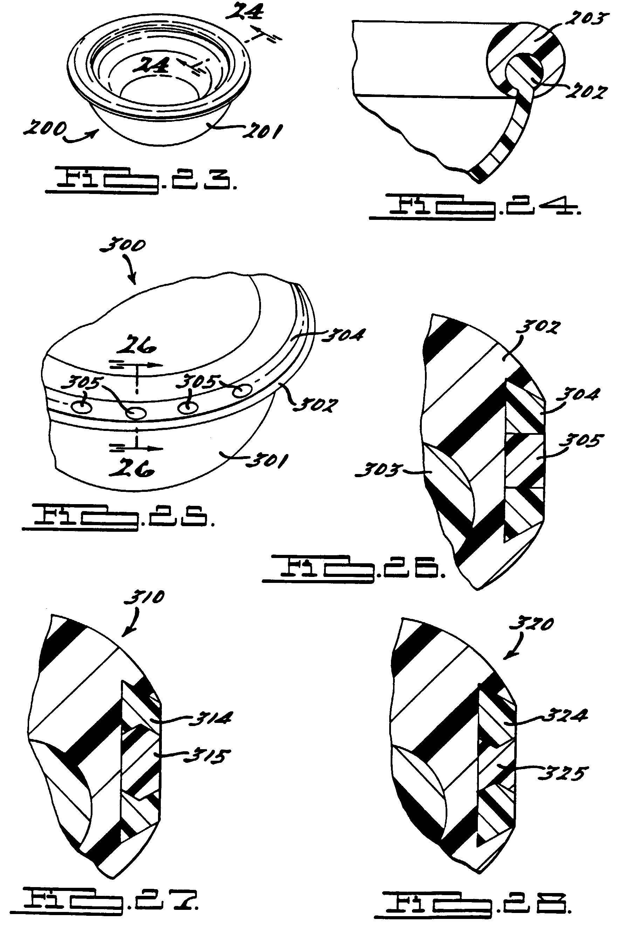

Figure 5 illustrates another preferred embodiment of a vaginal device of this invention vaginal device 100 which is analogous to vaginal device 10 except that groove 104 extends around the end face to retain annular ring 104 therein.

Now referring to Figures 6 and 7, another alternative embodiment of the present invention is shown and indicated generally by the numeral 60. Vaginal device 60 is a cervical cap having a dome portion 62 the open end of which terminates in an annular, bulbous edge 64. Bulbous edge 64 of dome portion 62 is adapted to snap-fit into rim 66. Thus, vaginal device 60 comprises a first part dome 62 and a second part rim 66. Rim 66 has an annular groove 68 adapted to receive and snappingly hold enlarged edge 64 of dome 62 to retain the two parts together.

Figures 8 and 9 illustrate another alternative embodiment of the present invention wherein a vaginal device 70 has a dome 71 and an annular rim 72 having an annular ring 73 snap-fit therein. Annular ring 73 in turn comprises a plurality of buttons 74 press fit therein. Thus a cervical device 70 comprises a plurality of parts including dome 71, annular ring 73 and a plurality of buttons 74. Annular ring 73 and each of buttons 74 can comprise the same of different biologically active materials.

Figures 10 and 11 are sectional views analogous to Figure 9 but showing alternative arrangements for means for retaining buttons 74 in annular ring 73. Thus in the embodiment shown in Figure 10 a button 75 has shoulders 76 which fit in corresponding recesses 77 in annular ring 78 to provide a snap fit therein.

In Figure 11, annular ring 92 which is analogous to annular ring 72 of Figure 8, carries annular ring 93 which is snap-fit therein. A plurality of buttons 94 are in turn snap-fit into annular ring 93. Thus, as is shown in Figure 11, annular ring 93 has shoulders 95 which fit into corresponding recesses 96 in ring-like buttons 94 to provide a snap-fit for buttons 94 into ring 93.

Figure 12 shows a vaginal device 100 which is a vaginal ring comprising a first part 101 and a second part 102. End 103 of part 101 snap-fits into end 104 of part 102 as is illustrated in Figure 13.

Figure 14 shows a vaginal device 110 having a plurality of buttons 111 fit into strip 112.

Figure 15 shows a vaginal device 120 comprising a first part 121 and a second pan 122 each of which are in strip form. Part 121 has a recess 123 into which snappingly fits a projection 124 of part 122.

Figure 16 shows a three part vaginal device 130 comprising a first strip 131, a second strip 132 and a third strip 133. First strip 131 has a recess 134 into which snappingly fits projection 135 of strip 132. Strip 132 also has a projection 136 at the end opposite thereof from projection 135. Projection 136 fits into a corresponding recess 137 and strip 133.

Figure 17 shows a vaginal ring 140 comprising a first annular piece 141 and a second annular piece 142. As is best shown in Figure 18, first annular piece 141 has a projection 143 which snappingly fits into a corresponding recess 144 in annular piece 142.

Now referring to Figures 19 and 20, two alternative embodiments of vaginal devices of the present invention are shown and indicated as vaginal device 150 and 160, respectively. Vaginal device 150, as shown in Figure 19, comprises two disc halves 151 and 152. Vaginal disc half 151 has a recess 153 into which snappingly fits projection 154 of disc half 152. The vaginal device 160 as shown in Figure 20 comprises three parts, vaginal disc third 161, vaginal disc third 162 and vaginal disc third 163. The vaginal disc thirds have recesses 164, 165, and 166 into which snappingly fit projections 167, 168 and 169.

Figure 21 illustrates a vaginal device 170 which is a vaginal ring comprising four parts or segments 171, 172, 173 and 174. The opposite ends of each segment are provided with means for snappingly attaching to the adjacent opposite end of another segment. Thus segment 171 has one end having recess 175 therein which snappingly receives projection 176 of segment 172. The other end of segment 171 has projection 178 therein which snappingly fits into recess 177 of segment 174. Each of segments 172, 173 and 174 have corresponding means at each end to snappingly retrain adjacent ends of other segments.

Figure 22 illustrates a vaginal device 180 comprised of two parts 181 and 182 and suitable for use as a vaginal suppository. Length 181 snappingly engages length 182.

Figures 23 - 28 illustrate alternative embodiments of vaginal devices of the present invention which are diaphragms. The vaginal device 200 of Figure 23 has a dome portion 201 with an enlarged rim 202 which is snappingly retained within an annular ring 203.

The vaginal device 300 shown in Figure 25 is a diaphragm having a dome 301 with an annular rim 302 press fit over enlarged annular edge 303. Annular rim 302 in turn carries snappingly fit therein an annular ring 304 into which are press fit a plurality of buttons 305.

Figures 27 and 28 show alternative press fit arrangements. In Figure 27 vaginal device 310 which is analogous to vaginal device 300 has an annular ring 314 which carries a plurality of buttons 315. In Figure 28 vaginal device 320 has an annular ring 324 which carries a plurality of buttons 325.

From the above description of variations of the present invention it will be appreciated that the present invention is subject to substantial modification and variation by one skilled in the art within the scope of the present invention. Therefore, it is intended that the present invention be limited only by the scope of the appended claims.

## Claims

1. A vaginal device (10,60,70,100,130,140,150,160,161,170,180,200, 300,310,320) having a first member (12,60,101,131,141,151,162,171,181,201,301) and a second member (14,56,66,102,104,132,142,152,172,182,203,302), at least one of said members comprising a releasable biologically active material, characterised in that said members are interchangeably and selectively snap-fit together.

2. A vaginal device as in claim 1, wherein said first and second members are comprised of a silicone based material.

3. A vaginal device as in claim 1 or claim 2, wherein said biologically active material is a medicament or a spermicide.

4. A vaginal device as in any one of said preceding claims, wherein both of said first and second members comprise a releasable biologically active material.

5. A vaginal device as in claim 4, wherein said biologically active materials are different materials.

6. A vaginal device as in any one of the preceding claims, wherein said device (10,60) is a cervical cap and said first member is a form assuming pliable dome (12,62,71,201) with a flexible annular rim (18,72,202) and said second member (14,66,73,203) is retained in or over said rim.

7. A vaginal device as in claim 6, wherein said second member (14,73) is an annular ring snap-fit into said annular rim (18,72).

8. A vaginal device as claimed in claim 7, wherein said annular ring (14,73) has a trapezoidal, circular or rectangular cross-sectional shape.

9. A vaginal device as claimed in claim 6, wherein said second member (73) comprises a button press fit into said rim (72).

10. A vaginal device as in claim 6, wherein said second member (93) has a third member (94) retained therein, said third member comprising a releasable, biologically active material.

11. A vaginal device as in claim 10, wherein said third member (94) is a button press fit into said second member.

12. A vaginal device (100,130) as in any one of claims 1 to 5, wherein said device is a vaginal ring in the shape of an elongated belt with opposite first and second ends (103,104) which snap-fit together to form a ring structure, said first end (103) comprising the first member (101,131,170) and said second end (104) comprising the second member (102,132,171).

13. A vaginal device as in claim 12, wherein said vaginal ring comprises a third member (111) press fit into an aperture in one of said first and second members (101,102).

14. A vaginal device as in claim 12, wherein said vaginal ring comprises a third elongated belt (133) snap-fit between said first and second ends.

15. A vaginal device as in any one of claims 1 to 5, wherein at least one of said first and second members (101,102,131,132) is an elongated strip.

16. A vaginal device as in claim 15, wherein said first member is an elongated strip, said second member comprises a biologically active material and is a button press fit into said first member.

## Patentansprüche

1. Eine Vaginal-Vorrichtung (10, 60, 70, 100, 130, 140, 150, 160, 161, 170, 180, 200, 300, 310, 320) bestehend aus einem ersten Teil (12, 60, 101, 131, 141, 151, 162, 171, 181, 201, 301) und einem zweiten Teil (14, 56, 66, 102, 104, 132, 142, 152, 172, 182, 203, 302), wobei wenigstens eines dieser Teile einen freisetzbaren biologisch aktiven Stoff enthält,
dadurch gekennzeichnet,
daß die genannten Teile miteinander austauschbar und selektiv durch Einschnappen verbindbar sind.

2. Eine Vaginal-Vorrichtung nach Anspruch 1, wobei das erste und das zweite Teil aus einem Material auf Silikonbasis bestehen.

3. Eine Vaginal-Vorrichtung nach einem der Ansprüche 1 oder 2, wobei der biologisch aktive Stoff ein Medikament oder ein Spermizid ist.

4. Eine Vaginal-Vorrichtung nach einem der vorangegangenen Ansprüche, wobei sowohl das erste als auch das zweite Teil einen freisetzbaren biologisch aktiven Stoff enthalten.

5. Eine Vaginal-Vorrichtung nach Anspruch 4, wobei es sich bei den biologisch aktiven Stoffen um unterschiedliche Stoffe handelt.

6. Eine Vaginal-Vorrichtung nach einem der vorangegangenen Anprüche, wobei die Vorrichtung (10, 60) eine Cervical-Kappe ist, und das erste Teil eine die Form annehmende biegsame Haube (12, 62, 71, 201) mit einer flexiblen ringförmigen Krempe (18, 72, 202) ist, und das zweite Teil (14, 66, 73, 203) in oder über dieser Krempe gehalten wird.

7. Eine Vaginal-Vorrichtung nach Anspruch 6, wobei das zweite Teil (14, 73) ein kreisförmiger Ring ist, der in die ringförmige Krempe (18, 72) eingeschnappt ist.

8. Eine Vaginal-Vorrichtung nach Anspruch 7, wobei der kreisförmige Ring (14, 73) einen trapezoidförmigen, kreisförmigen oder rechteckförmigen Querschnitt hat.

9. Eine Vaginal-Vorrichtung nach Anspruch 6, wobei das zweite Teil (73) druckknopfartig in der Krempe (72) befestigt ist.

10. Eine Vaginal-Vorrichtung nach Anspruch 6, wobei das zweite Teil (93) ein in diesem gehaltenes drittes Teil (94) aufweist und das dritte Teil einen freisetzbaren, biologisch aktiven Stoff enthält.

11. Eine Vaginal-Vorrichtung nach Anspruch 10, wobei das dritte Teil (94) druckknopfartig in dem zweiten Teil befestigt ist.

12. Eine Vaginal-Vorrichtung (100, 130) nach einem der Ansprüche 1 bis 5, wobei die Vorrichtung ein Vaginal-Ring ist, der die Form eines langgestreckten Gurtes mit sich gegenüberliegenden ersten und zweiten Enden (103, 104), die ineinander eingeschnappt sind, um so eine Ringkonstruktion zu bilden, aufweist, wobei das erste Ende (103) das erste Teil (101, 131, 170) und das zweite Ende (104) das zweite Teil (102, 132, 171) aufweist.

13. Eine Vaginal-Vorrichtung nach Anspruch 12, wobei der Vaginal-Ring ein drittes Teil (111) umfaßt, das in eine Öffnung in dem ersten oder dem zweiten Teil (101, 102) eingeschnappt ist.

14. Eine Vaginal-Vorrichtung nach Ansprach 12, wobei der Vaginal-Ring einen dritten langgestreckten Gurt (133) umfaßt, der zwischen dem ersten und dem zweiten Ende eingeschnappt ist.

15. Eine Vaginal-Vorrichtung nach einem der Ansprüche 1 bis 5, wobei mindestens eines von erstem und zweitem Teil (101, 102, 131, 132) ein langgestrecktes Band ist.

16. Eine Vaginal-Vorrichtung nach Anspruch 15, wobei das erste Teil ein langgestrecktes Band ist und das zweite Teil biologisch aktives Material enthält und druckknopfartig mit dem ersten Teil verbunden ist.

## Revendications

1. Dispositif vaginal (10, 60, 70, 100, 130, 140, 150, 160, 161, 170, 180, 200, 300, 310, 320) comportant un premier élément (12, 60, 101, 131, 141, 151, 162, 171, 181, 201, 301) et un deuxième élément (14, 56, 66, 102, 104, 132, 142, 152, 172, 182, 203, 302), au moins un desdits éléments comprenant une substance bioactive libérable, caractérisé en ce que lesdits éléments sont ajustés par enclenchement de façon interchangeable et sélective.

2. Dispositif vaginal selon la revendication 1, où lesdits premier et deuxième éléments sont en un matériau à base de silicone.

3. Dispositif vaginal selon la revendication 1 ou la revendication 2, où ladite substance bioactive est un médicament ou un spermicide.

4. Dispositif vaginal selon l'une quelconque des revendications précédentes, où lesdits premier et deuxième éléments comprennent l'un et l'autre une substance bioactive libérable.

5. Dispositif vaginal selon la revendication 4, où lesdites substances bioactives sont des substances différentes.

6. Dispositif vaginal selon l'une quelconque des revendications précédentes, où ledit dispositif (10, 60) est une cape cervicale et ledit premier élément est un dôme pliable assumant une forme (12, 62, 71, 201) comportant un bord annulaire flexible (18, 72, 202) et ledit deuxième élément (14, 66, 73, 203) est retenu dans ou sur ledit bord.

7. Dispositif vaginal selon la revendication 6, où ledit deuxième élément (14, 73) est une bague annulaire ajustée par enclenchement dans ledit bord annulaire (18, 72).

8. Dispositif vaginal selon la revendication 7, où ladite bague annulaire (14, 73) a une section transversale de forme trapézoïdale, circulaire ou rectangulaire.

9. Dispositif vaginal selon la revendication 6, où ledit deuxième élément (73) comprend un bouton ajusté par pression dans ledit bord (72).

10. Dispositif vaginal selon la revendication 6, où ledit deuxième élément (93) contient un troisième élément (94) retenu à l'intérieur, ledit troisième élément comprenant une substance bioactive libérable.

11. Dispositif vaginal selon la revendication 10, où ledit troisième élément (94) est un bouton ajusté par pression dans ledit deuxième élément.

12. Dispositif vaginal (100, 130) selon l'une quelconque des revendications 1 à 5, où ledit dispositif est une bague vaginale ayant la forme d'une bande allongée comportant des première et deuxième extrémités (103, 104) opposées qui s'ajustent par enclenchement pour former une structure annulaire, ladite première extrémité (103) comprenant le premier élément (101, 131, 170) et ladite deuxième extrémité (104) comprenant le deuxième élément (102, 132, 171).

13. Dispositif vaginal selon la revendication 12, où ladite bague vaginale comprend un troisième élément (111) ajusté par pression dans une ouverture de l'un desdits premier ou deuxième éléments (101, 102).

14. Dispositif vaginal selon la revendication 12, où ladite bague vaginale comprend une troisième bande allongée (133) ajustée par pression entre lesdites première et deuxième extrémités.

15. Dispositif vaginal selon l'une quelconque des revendications 1 à 5, où au moins un desdits premier et deuxième éléments (101, 102, 131, 132) est un ruban allongé.

16. Dispositif vaginal selon la revendication 15, où ledit premier élément est un ruban allongé, ledit deuxième élément comprend une substance bioactive et est un bouton ajusté par pression dans ledit premier élément.
